# EUROPÄISCHE PATENTSCHRIFT

(11) **EP 1 796 619 B1**
(45) Veröffentlichungstag und Bekanntmachung des Hinweises auf die Patenterteilung: **18.05.2016**
(21) Anmeldenummer: 05789617.7
(22) Anmeldetag: 22.09.2005
(51) Int. Cl.: A61K 8/27, A61K 8/29, A61K 8/46, A61K 8/49, A61Q 17/04, A61K 8/06

(54) **O/W-EMULSION MIT ANORGANISCHEN UV-LICHTSCHUTZFILTERPIGMENTEN UND ALKYLSUFAT**
O/W EMULSION COMPRISING INORGANIC UV FILTER PIGMENTS AND ALKYL SULFATE
EMULSION HUILE DANS L'EAU CONTENANT DES PIGMENTS FILTRANTS DE PROTECTION SOLAIRE ANTI-UV INORGANIQUES ET DU SULFATE D'ALKYLE

(30) Priorität: 27.09.2004 DE 102004047283
(43) Veröffentlichungstag der Anmeldung: 20.06.2007
(73) Patentinhaber: Beiersdorf AG, 20253 Hamburg (DE)
(72) Erfinder: NISSEN, Bente, 20144 Hamburg (DE); LERG, Heike, 22303 Hamburg (DE); MÜLLER, Anja Sabine, 26789 Leer (DE)
(86) Internationale Anmeldenummer: PCT/EP2005/054740
(87) Internationale Veröffentlichungsnummer: WO 2006/034985

(56) Entgegenhaltungen:
- DE-A1- 10 260 877
- US-A- 5 747 012
- US-A1- 2003 228 334
- US-A1- 2004 081 629
- ANONYMOUS: "Symmetrical Triazine derivatives" IP.COM, [Online] Nr. ipcom000031257d, 20. September 2004 (2004-09-20), Seiten 1-127, XP002360102 Gefunden im Internet: URL:https://priorart.ip.com/download/IPCOM 000031257D/IPCOM000031257D.pdf> [gefunden am 2005-12-19]

## Beschreibung

Die vorliegende Erfindung betrifft eine kosmetische O/W-Emulsion enthaltend Alkylsulfat, anorganische UV-Lichtschutzfilterpigmente sowie W/O-Emulgatoren, die dadurch gekennzeichnet ist, dass die Zubereitung einen Lichtschutzfaktor von mindestens 15 aufweist.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie der Anlage 7 der deutschen Kosmetikverordnung zusammengefasst.

Eine besondere Form von UV-Lichtschutzfiltersubstanzen stellen die anorganischen Mikropigmente dar. Die UV-Schutzwirkung der Mikropigmente beruht auf den physikalischen Effekten der Reflexion und Lichtstreuung. In kosmetischen Zubereitungen werden als anorganische Mikropigmente meist Pigmente aus Titandioxid, Zinkoxid oder Mischoxiden mit zum Beispiel Eisenoxiden eingesetzt.

Die Vorteile von anorganischen Mikropigmenten als UV-Filtersubstanz in kosmetischen Zubereitungen liegen vor allem darin begründet, dass die Pigmente im Gegensatz zu organischen Lichtschutzfiltern, physikalisch und chemisch besonders stabil sind. Sie neigen selbst bei starker UV-Strahlung nicht zur Zersetzung oder zu Photoreaktionen. Aufgrund der Tatsache, dass es sich bei den Pigmenten um Feststoffe handelt, besteht keine Gefahr einer übermäßigen Penetration der Filter in die Haut. Das Auftreten von allergischen Reaktionen ist damit ausgeschlossen.

Nachteilig am Stande der Technik ist jedoch der Umstand, dass anorganische Mikropigmente nur schwer stabil in kosmetische Zubereitungen einzuarbeiten sind. Insbesondere wenn Mikropigmente in höheren Konzentrationen (Konzentrationen über 10 Gewichts-%, bezogen auf das Gesamtgewicht der Zubereitung) eingesetzt werden, bilden sie relativ schnell Pigment-Agglomerate, die aus der Zubereitung ausfallen. Die Lagerstabilität ist also gering. Auch bei der Anwendung auf der Haut führen höhere Konzentrationen von anorganischen Mikropigmenten schnell zur Agglomeration die in Form von weißen Flecken und Streifen ("weißeln") auf der Haut sichtbar und vom Anwender als unästhetisch empfunden werden. Ferner führt die Bildung der Agglomerate zu einer Veränderung im Absorptionsverhalten der Pigmente während der Anwendung. Größere Agglomerate absorbieren überwiegend im Bereich des sichtbaren Lichtes (daher das "weißeln") sowie im UV-A-Bereich, während kleinere Partikel ihr Absorptionsmaximumm im UV-B-Bereich besitzen. Darüber hinaus ist die UV-Lichtschutzwirkung von größeren Teilchen deutlich geringer als die von Mikropigmenten. Nach dem Stande der Technik ist es also nahezu unmöglich, kosmetische UV-Lichtschutzzubereitungen auf der Basis von anorganischen Mikropigmenten herzustellen, die während der Dauer ihrer Anwendung ein stabiles und ausgewogenes Absorptionsspektrum aufweisen.

Nach dem Stand der Technik lassen sich diese Probleme allein dadurch begrenzen, dass als kosmetische Grundlage der Sonnenschutzzubereitung eine Wasser-in-Öl-Emulsion (W/O-Emulsion) eingesetzt werden. W/O-Emulsionen weisen aber, da ihre äußere Phase von der Ölphase gebildet wird, in der Regel ein eher ölig-fettiges Hautgefühl auf. W/O-Emulsionen mit einem erhöhten Gehalt an anorganischen Mikropigmenten wirken darüber hinaus zäh und lassen sich nur schwer auf der Haut verteilen. Um die Verteilbarkeit solcher Zubereitungen zu verbessern, müssen den Formulierungen Silikonöle zugesetzt werden, was häufig nicht erwünscht ist.

In die kosmetisch-sensorisch attraktiveren Öl-in-Wasser-Emulsionen (O/W-Emulsionen) ließen sich bisher keine größeren Mengen an anorganischen Mikropigmenten einarbeiten, ohne dass die beschriebenen Nachteile zu vermeiden gewesen wären. Es ließen sich allenfalls Zubereitungen mit geringem Lichtschutzfaktor stabil formulieren.

Aufgabe der vorliegenden Erfindung war es daher, die Nachteile des Standes der Technik zu beseitigen und kosmetische Lichtschutzzubereitungen mit hohem Lichtschutzfaktor auf der Basis von Öl-in-Wasser-Emulsionen (O/W-Emulsionen) zu entwickeln, deren UV-Filtersystem auf anorganischen Mikropigmenten basiert. Diese Zubereitungen sollten insbesondere lager- und transportstabil sein und eine Agglomeration der Mikropigmente während der Lagerung und der Anwendung wirkungsvoll unterdrücken. Die Formulierungen sollten ferner bei Ihrer Anwendung auf der Haut ein über einen längeren Anwendungszeitraum stabiles Absorptionsspektrum mit einer ausgewogenen UV-A/UV-B-Absorptionsbalance aufweisen.

Überraschend gelöst werden die Aufgaben durch eine kosmetische O/W-Emulsion gemäß Anspruch 1.

Erfindungsgemäß bedeutet "mindestens 10 Gewichts%", dass die Menge an anorganischen UV-Lichtschutzfilterpigmenten größer oder gleich 10 Gewichts-% beträgt. Entsprechendes gilt auch für die Angaben zum Lichtschutzfaktor.

Zwar weisen beschreiben die DE 10162842, DE19923645, DE19933461, DE10040481, DE10063345, DE10100409, DE10008896, DE10161884, DE10226353, DE10140546, DE10135024, DE10154111, DE10260877, DE10141473, DE10141474, DE10247357, EP1302197, EP1093798, EP1093797, EP1093796. EP1064922, EP893119 und EP1068866 kosmetische Zubereitungen mit einem Gehalt an anorganischen UV-Filterpigmenten, doch konnten diese Schriften nicht den Weg zur vorliegenden Erfindung weisen.

Darüber hinaus kennt der Fachmann die US 2004/081629, DE 10260877, US 5747012, US 2003/228334 sowie Anonymus: "Symmetrical Triazine derivatives", IP.COM Nr. 000031257d, 20. September 2004, die ebenfalls nicht den Weg zur vorliegenden Erfindung weisen konnten.

Die erfindungsgemäßen Zubereitungen weisen in Ihren bevorzugten Ausführungsformen ein thixotropes Verhalten auf, d.h. das sie unter der Einwirkung einer Scherkraft (z.B. beim Verreiben auf der Haut) dünnflüssiger werden und sich damit leichter verteilen lassen.

Überraschend und für den Fachmann unerwartet steigt bei den erfindungsgemäßen Zubereitungen der Lichtschutzfaktor bei einer Erhöhung der Einsatzmenge an UV-Lichtschutzfilterpigmenten besonders stark an. So führt eine Erhöhung des Anteils an partikulären anorganischen UV-Lichtschutzfilterpigmenten um 1 Gewichts-% zu einer Erhöhung des Lichtschutzfaktors von über einer Einheit (z.B. führen 17 Gewichts-% Mikropigmentanteil zu einem Lichtschutzfaktor 23,4).

### Messmethoden

Bestimmung des erfindungsgemäßen Lichtschutzfaktors erfolgte wie in der DIN 67501 Sep. 1999 angeben, nach der gemessen wird.

Die Partikelgröße der anorganischen UV-Lichtschutzfilterpigmente wurde mittels Rasterelektronenmikroskopie (REM) bestimmt.

Die im Rahmen der vorliegenden Schrift aufgeführten Viskositätswerte der Zubereitungen und Einzelsubstanzen wurden mit Hilfe eines Viskosimeters des Typs Viskotester VT 02 der Gesellschaft Haake ermittelt (Temperatur: 20°C, Spindeldurehmesser 24 mm, Rotorgeschwindigkeit 62 1/min nach 30s).

Es ist erfindungsgemäß bevorzugt, das erfindungsgemäße Alkylsulfat in einer Konzentration von 0,05 bis 1,5 Gewichts-% und besonders bevorzugt in einer Konzentration von 0,1 bis 1,0 Gewichts-%, jeweils bezogen auf das Gesamtgewicht der Zubereitung, einzusetzen.

Erfindungsgemäß besonders bevorzugte Ausführungsformen der vorliegenden Erfindung sind dadurch gekennzeichnet, dass als Alkylsulfat Cetostearylsulfat eingesetzt wird.

Es ist erfindungsgemäß bevorzugt, Fettalkohol und/oder Glycerinmonostearat und/oder Sorbitanstearat in einer Gesamtkonzentration von 0,5 bis 8,0 Gewichts-% und besonders bevorzugt in einer Konzentration von 1,0 bis 6,0 Gewichts-%, jeweils bezogen auf das Gesamtgewicht der Zubereitung, einzusetzen.

Als erfindungsgemäß vorteilhafte Fettalkohole können beispielsweise Cetylalkohol, Stearylalkohol, Behenylalkohol, Isostearylalkohol oder deren Mischungen eingesetzt werden, wobei eine Mischung aus Cetylalkohol und Stearylalkohol erfindungsgemäß bevorzugt ist.

Erfindungsgemäß besonders bevorzugt ist es eine Mischung aus Cetostearylalkohol und Glycerinmonostearat einzusetzen.

Bevorzugt im Sinne der vorliegenden Erfindung werden anorganische UV-Lichtschutzfilterpigmente in einer Menge von mindestens 15 Gewichts-%, bezogen auf das Gesamtgewicht der Zubereitung, in dieser eingesetzt.

Es ist dabei erfindungsgemäß von Vorteil, wenn die anorganischen UV-Lichtschutzfilterpigmente gewählt werden aus der Gruppe der folgenden Pigmente:

Die Titandioxid- Pigmente können sowohl in der Kristallmodifikation Rutil als auch Anatas vorliegen und können im Sinne der vorliegenden Erfindung vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtung können im Sinne der vorliegenden Erfindung auch Wasser enthalten.

Beschriebene beschichtete und unbeschichtete Titandioxide können im Sinne vorliegender Erfindung auch in Form kommerziell erhältlicher öliger oder wäßriger Vordispersionen zur Anwendung kommen. Diesen Vordispersionen können vorteilhaft Dispergierhilfmittel und/oder Solubilisationsvermittler zugesetzt sein.

Die erfindungsgemäßen Titandioxide zeichnen sich durch eine Primärpartikelgröße zwischen 10 nm bis 200 nm aus, wobei Partikelgrößen von 10 nm bis 100 nm erfindungsgemäß bevorzugt sind.

| **Handelsname** | **Coating** | **zusätzliche Bestandteile der Vordispersion** | **Hersteller** |
|---|---|---|---|
| MT-100TV | Aluminiumhydroxid Stearinsäure | - | Tayca Corporation |
| MT-100Z | Aluminiumhydroxid Stearinsäure | - | Tayca Corporation |
| MT-100F | Stearinsäure Eisenoxid | - | Tayca Corporation |
| MT-500SAS | Alumina, Silica Silikon | - | Tayca Corporation |
| MT-100AQ | Silica Aluminiumhydroxid Alginsäure | - | Tayca Corporation |
| Eusolex T-2000 | Alumina Simethicone | - | Merck KgaA |
| Eusolex TS | Alumina, Stearinsäure | - | Merck KgaA |
| Eusolex T-AVO | Silica | | Merck KgaA |
| Titandioxid P25 | None | - | Degussa |
| Titandioxid T805 (Uvinul TiO₂) | Octyltrimethylsilan | - | Degussa |
| UV-Titan X170 | Alumina Dimethicone | - | Kemira |
| UV-Titan X161 | Alumina, Silica Stearinsäure | - | Kemira |
| Tioveil AQ 10PG | Alumina Silica | Wasser Propylenglycol | Solaveil Uniquema |
| Mirasun TIW 60 | Alumina Silica | Wasser | Rhone-Poulenc |
| Titandioxid T-817 (Eisen/TitanMisch oxid) | Eisenoxid | | Degussa |

Im Sinne der vorliegenden Erfindung sind besonders bevorzugte Titandioxide das MT-100 Z und MT-100 TV von Tayca Corporation, Eusolex T-2000 und Eusolex T-AVO von Merck und das Titandioxid T 805 von Degussa und das Eisen/Titandmischoxid Titandioxid T817 von Degussa.

Zinkoxide können im Sinne der vorliegenden Erfindung auch in Form kommerziell erhältlicher öliger oder wäßriger Vordispersionen zur Anwendung kommen. Erfindungsgemäß geeignete Zinkoxidpartikel und Vordispersionen von Zinkoxidpartikeln zeichnen sich durch eine Primärpartikelgröße von < 300 nm aus und sind unter folgenden Handelsbezeichnungen bei den aufgeführten Firmen erhältlich:

| **Handelsname** | **Coating** | **Hersteller** |
|---|---|---|
| Z- Cote HP1 | 2% Dimethicone | BASF |
| Z- Cote | / | BASF |
| ZnO NDM | 5% Dimethicone | H&R |
| MZ 707M | 7% Dimethicone | M. Tayca Corp. |
| Nanox 500 | / | Elementis |
| ZnO Neutral | / | H&R |

Besonders bevorzugte Zinkoxide im Sinne der Erfindung sind das Z-Cote HP1 von der Firma BASF und das Zinkoxid NDM von der Firma Haarmann & Reimer.

Besonders vorteilhaft ist es hydrophob modifizierte Titandioxidpigmente mit amphiphilen Zinkoxidpigmenten zu kombinieren.

Es ist bevorzugt im Sinne der vorliegenden Erfindung, wenn die Zubereitung einen Lichtschutzfaktor von mindestens 20 aufweist.

Erfindungsgemäß vorteilhafte Ausführungsformen der vorliegenden Erfindung sind ferner dadurch gekennzeichnet, dass die Zubereitungen eine Viskosität von 200 bis 15000 mPas und besonders bevorzugt eine Viskosität von 1500 bis 12000 mPas aufweisen.

Vorteilhafte Ausführungsformen der vorliegenden Erfindung können mit weniger als 10% Silikonölen formuliert werden, insbesondere weniger als 5% Silikonöl, oder gänzlich frei von Silikonölen sein.

Vorteilhafte Ausführungsformen der vorliegenden Erfindung können mit weniger als 10% Mineralölen formuliert werden, insbesondere weniger als 5% Mineralöl, oder gänzlich frei von Mineralölen sein.

Es ist erfindungsgemäß von Vorteil, wenn das Verhältnis von Alkylsulfat zur Gesamtmenge an partikulären anorganischen UV-Lichtschutzfilterpigmenten von 1 : 7 bis 0,1 : 60 beträgt und erfindungsgemäß bevorzugt, wenn dass Verhältnis von Alkylsulfat zur Gesamtmenge an partikulären anorganischen UV-Lichtschutzfilterpigmenten von 1 : 20 bis 0,1 : 30 beträgt.

Erfindungsgemäß vorteilhafte Ausführungsformen der vorliegenden Erfindung sind dadurch gekennzeichnet, dass die Zubereitung einen oder mehrere W/O-Emulgatoren in einer Gesamtkonzentration von 0,2 bis 1,5 Gewichts-%, bezogen auf das Gesamtgewicht der Zubereitung, enthält.

Es ist erfindungsgemäß vorteilhaft, wenn die erfindungsgemäße Zubereitung frei ist von in der Öl- oder Wasserphase löslichen oder flüssigen organischen UV-Lichtschutzfiltern. Die erfindungsgemäße Emulsion kann aber auch ein oder mehrere der in der Kosmetik bekannten, in der Öl- oder Wasserphase löslichen oder flüssigen organischen UV-Lichtschutzfilter enthalten.

Vorteilhafte Ausführungsformen der vorliegenden Erfindung sind dadurch gekennzeichnet, dass die Zubereitung partikuläre organische Lichtschutzfilter gewählt aus der Gruppe 2,2'-Methylen-bis-(6-(2H-benzotriazol-2-yl)-4-(1,1,3,3-tetramethylbutyl)-phenol); 2,4,6-Tris-(biphenyl)-1,3,5-triazin (insbesondere 2,4,6-Tribiphenyl-4-yl-1,3,5-triazin); 2,4,6-Tris-(terphenyl)-1,3,5-triazin, enthalten.

Die erfindungsgemäßen Zubereitungen können ferner vorteilhaft auch Selbstbräunungssubstanzen enthalten, wie beispielsweise Dihydroxyacteon und/oder Melaninderivate in Konzentrationen von 1 Gew.-% bis zu 10 Gew.-%, bezogen auf das Gesamtgewicht der Zubereitung.

Ferner vorteilhaft können die erfindungsgemäßen Zubereitungen auch Repellentien zum Schutz vor Mücken, Zecken und Spinnen und dergleichen enthalten. Vorteilhaft sind z. B. N,N-Diethyl-3-methylbenzamid (Handelsbezeichnung: Meta-delphene, "DEET"), Dimethylphtalat (Handelsbezeichnung: Palatinol M, DMP), 1-Piperidincarbonsäure-2-(2-hydroxyethyl)-1-methylpropylester sowie insbesondere 3-(N-n-Butyl-N-acetyl-amino)-propionsäureethylester (unter dem Handelsnamen Insekt Repellent® 3535 bei der Fa. Merck erhältlich). Die Repellentien können sowohl einzeln als auch in Kombination eingesetzt werden.

Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Es ist vorteilhaft im Sinne der vorliegenden Erfindung, wenn die erfindungsgemäße Zubereitung ein oder mehrere Feuchthaltemittel in einer Gesamtkonzentration von 0,1 bis 20 Gewichts-% und bevorzugt in einer Gesamtkonzentration von 0,5 bis 10 Gewichts-%, jeweils bezogen auf das Gesamtgewicht der Zubereitung, enthält.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9).

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder -monobutylether, Di-ethylenglykolmonomethyl- oder -monoethylether und analoge Produkte, Polymere, Schaumstabilisatoren, Elektrolyte, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Die Ölphase der O/W-Emulsion der erfindungsgemäßen Zubereitung wird vorteilhaft gewählt aus der Gruppe der polaren Öle, beispielsweise aus der Gruppe der Lecithine und der Fett-säuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol* CC bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon (*Hallstar AB*) und/oder Diethylhexylnaphthalat (*Hallbrite TQ oder Corapan TQ von H&R*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Es ist erfindungsgemäß vorteilhaft, wenn der Silikonölgehalt der erfindungsgemäßen Zubereitung weniger als 5 Gewichts-%, bezogen auf das Gesamtgewicht der Zubereitung, beträgt.

Auch ist es erfindungsgemäß vorteilhaft, wenn der Mineralölgehalt der erfindungsgemäßen Zubereitung weniger als 5 Gewichts-%, bezogen auf das Gesamtgewicht der Zubereitung, beträgt.

Die erfindungsgemäßen Zubereitungen können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern:
(a) Siloxanelastomere, welche die Einheiten R₂SiO und RSiO_{1,5} und/oder R₃SiO_{0,5} und/oder SiO₂ enthalten,
   wobei die einzelnen Reste R jeweils unabhängig voneinander Wasserstoff, C₁₋₂₄-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten R₂SiO zu RSiO_{1,5} aus dem Bereich von 1 : 1 bis 30 : 1 gewählt wird;
(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,
   wobei die verwendeten Mengenateile so gewählt werden, dass die Menge des Wasserstoffes des Organopolysiloxans (1) oder der ungesättigten aliphatischen Gruppen des Organopolysiloxans (2)
   - im Bereich von 1 bis 20 mol-% liegt, wenn das Organopolysiloxan nicht zyklisch ist und
   - im Bereich von 1 bis 50 mol-% liegt, wenn das Organopolysiloxan zyklisch ist.

Vorteilhaft im Sinne der vorliegenden Erfindung liegen das oder die Siloxanelastomere in Form sphärischer Puder oder in Form von Gelen vor.

Erfindungsgemäß vorteilhafte in Form sphärischer Puder vorliegende Siloxanelastomere sind die mit der INCl-Bezeichnung Dimethicone / Vinyl Dimethicone Crosspolymer, beispielsweise das von DOW CORNING unter der Handelsbezeichnungen DOW CORNING 9506 Powder erhältliche.

Besonders bevorzugt ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin, Kreatinin, Taurin und/oder ß-Alanin sowie 8-Hexadecen-1,16-dicarbonsäure (Dioic acid, CAS-Nummer 20701-68-2; vorläufige INCl-Bezeichnung Octadecendioic acid) und/oder Licochalcon A.

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zum Schutz vor ästhetisch unattraktiven Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

Die kosmetischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern oder Steigern des Schäumens, Farbstoffe, Pigmente, die eine färbende Wirkung haben, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Vorteilhaft im Sinne der vorliegenden Erfindung sind Zubereitungen zur Pflege der Haut: sie können dem kosmetischen Lichtschutz, ferner als Schminkprodukt in der dekorativen Kosmetik dienen.

Entsprechend ihrem Aufbau können kosmetische Zusammensetzungen im Sinne der vorliegenden Erfindung, beispielsweise verwendet werden als Hautschutzcräme, Tages- oder Nachtcreme usw. Es ist gegebenenfalls möglich und vorteilhaft, die erfindungsgemäßen Zusammensetzungen als Grundlage für pharmazeutische Formulierungen zu verwenden.

Es ist auch vorteilhaft im Sinne der vorliegenden Erfindung, kosmetische Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescremes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische Zubereitungen, die in der Form eines Sonnenschutzmittels vorliegen.

Zur Anwendung werden die erfindungsgemäßen kosmetischen Zubereitungen in der für Kosmetika üblichen Weise auf die Haut und/oder die Haare in ausreichender Menge aufgebracht.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele

| | **1** | **2** |
|---|---|---|
| Methylparaben | 0,3 | 0,1 |
| Propylparaben | 0,1 | 0,1 |
| Caprylsäure/Caprinsäure Triglyceride | 5 | 5 |
| Glycerin | 3 | 7,5 |
| Tocopherylacetat | 0,5 | |
| Cetylalkohol | 1,5 | 1,5 |
| Glycerylstearat SE | 0,5 | 2 |
| Sorbitanstearat | 0,5 | |
| Trinatrium EDTA | 1 | 1 |
| Phenoxyethanol | | 0,4 |
| Cetearylalkohol | 2,5 | |
| PEG-40 Rizinusöl | 0,6 | |
| Natriumcetearylsulfat | 0,3 | 0,2 |
| Hydrierte Cocos-Glyceride | 1 | 0,5 |
| Panthenol | 0 | 1,4 |
| Alkohol | 3 | 3 |
| Cetyl PEG/PPG-10/1 Dimethicone | 0 | 1 |
| Xanthangummi | 0,2 | 0,4 |
| Titandioxid T 805 | 9 | 9 |
| Eusolex T-AVO | | |
| Titandioxid VT 817 | | |
| C18-36 Acid Triglyceride | | 1 |
| Polyglyceryl-2 Dipolyhydroxystearat | 0 | 0 |
| PEG-30 Dipolyhydroxystearat | 0,5 | 0 |
| Zinkoxid NDM | 8 | 8 |
| Zinkoxid HP1 | | |
| Cetyldimethicon | 2 | |
| Phenyltrimethicon | | |
| Butylenglycol Dicaprylat/Dicaprat | | 10 |
| Mineralöl | 5 | |
| Dicaprylylcarbonat | 2 | |
| Dibutyladipat | | |
| Dicaprylylether | | 5 |
| Wasser | ad100 | ad100 |

| | **3** | **4** |
|---|---|---|
| Ethylparaben | 0 | 0 |
| Methylparaben | 0,3 | 0,3 |
| Propylparaben | 0,1 | 0,1 |
| Caprylsäure/Caprinsäure Triglyceride | 5 | 5 |
| Glycerin | 10 | 7,5 |
| Cetylalkhol | 0,5 | 2 |
| Glycerylstearat SE | | 1 |
| Trinatrium EDTA | | |
| Cetearylalkohol | 2,5 | |
| PEG-40 Rizinusöl | 0,4 | 0,2 |
| Natriumetearylsulfat | 0,2 | 0,4 |
| C12-15 Alkylbenzoate | 0 | 0 |
| Panthenol) | 0 | 0 |
| Butylmethoxydibenzoylmethan | 0 | 0 |
| Phenylbenzimidazolesulfonsäure | 0 | 0 |
| 2,2'-Methylen-bis-(6-(2H-benzotriazol-2-yl)-4-(1,1,3,3-tetramethylbutyl)-phenol) | 4 | |
| 2,4,6-Tris-(terphenyl)-1,3,5-triazin | | 3 |
| 2,4,6-Tris-(biphenyl)-1,3,5-triazin | 2 | 2 |
| C8-C16 Alkylpolyglycosid | 0,4 | 0,5 |
| VP/Hexadecene Copolymer | 0 | 0 |
| NaOH 45% | 0 | 0 |
| Alkohol | | |
| Cetyl PEG/PPG-10/1 Dimethicone | 0 | 1 |
| Xanthangummi | 0,4 | 0,2 |
| Ethylhexyltriazon | 0 | 0 |
| Titandioxid T805 | 10 | 10 |
| C18-36 Säuren Triglyceride | | |
| PEG-30 Dipolyhydroxystearat | 1 | 0 |
| Bis-Ethylhexyloxyphenol Methoxyphenyl Triazine | 0 | 0 |
| Diethylhexyl Butamido Triazone | 0 | 0 |
| Zinkoxid NDM | 8 | 5 |
| Butylene Glycol Dicaprylate/Dicaprate | 4 | 5 |
| Dicaprylylcarbonat | | 2 |
| Dibuyladipat | 5 | |
| Wasser | ad 100 | ad 100 |

## Patentansprüche

1. Kosmetische O/W-Emulsion enthaltend
a) Alkylsulfat in einer Konzentration von 0,05 bis 1,5 Gewichts-%
b) anorganische UV-Lichtschutzfilterpigmente in einer Menge von mindestens 10 Gewichts-%,
jeweils bezogen auf das Gesamtgewicht der Zubereitung,
**dadurch gekennzeichnet, dass** als anorganische UV-Lichtschutzfilterpigmente Mischungen aus Titandioxid- und Zinkoxidpigmenten eingesetzt werden,
dass die Zubereitung einen Lichtschutzfaktor von mindestens 15 aufweist und dass die ein oder mehrere W/O-Emulgatoren enthält. die gewählt werden aus der Gruppe der Verbindungen Polysiloxanpolyether-Polyalkan-Copolymeren , Polyglycerinpoly-12-hydroxystearat, oder PEG-30 Dipolyhydroxystearat.

2. Kosmetische Zubereitung nach Anspruch 1, **dadurch gekennzeichnet, dass** die Emulsion Fettalkohol und/oder Glycerinmonostearat und/oder Sorbitanstearat in einer Gesamtkonzentration von 0,5 bis 8,0 Gewichts-%, bezogen auf das Gesamtgewicht der Zubereitung, enthält.

3. Kosmetische Zubereitung nach einem der vorhergehenden Ansprüche, **dadurch gekennzeichnet, dass** das Verhältnis von Alkylsulfat zur Gesamtmenge an anorganischen UV-Lichtschutzfilterpigmenten von 1 : 7 bis 0,1 : 60 beträgt.

4. Kosmetische Zubereitung nach einem der vorhergehenden Ansprüche, **dadurch gekennzeichnet, dass** als Alkylsulfat Cetostearylsulfat eingesetzt wird.

5. Kosmetische Zubereitung nach einem der vorhergehenden Ansprüche, **dadurch gekennzeichnet, dass** die anorganischen UV-Lichtschutzfilterpigmente gewählt werden aus der Gruppe der Titandioxide mit einer Primärpartikelgröße zwischen 10 nm bis 200 nm und/oder der Zinkoxide mit einer Primärpartikelgröße von < 300 nm .

6. Kosmetische Zubereitung nach einem der vorhergehenden Ansprüche, **dadurch gekennzeichnet, dass** die Zubereitung einen oder mehrere W/O-Emulgatoren in einer Gesamtkonzentration von 0,2 bis 1,5 Gewichts-%, bezogen auf das Gesamtgewicht der Zubereitung, enthält.

7. Kosmetische Zubereitung nach einem der vorhergehenden Ansprüche, **dadurch gekennzeichnet, dass** die Zubereitung den partikulären organischen Lichtschutzfilter 2,2'-Methylen-bis-(6-(2H-benzotriazol-2-yl)-4-(1,1,3,3-tetramethylbutyl)-phenol) enthält.

8. Kosmetische Zubereitung nach einem der vorhergehenden Ansprüche, **dadurch gekennzeichnet, dass** die Zubereitung einen oder mehrere partikuläre organische Lichtschutzfilter gewählt wird aus der Gruppe 2,4,6-Tris-(biphenyl)-1,3,5-triazin; 2,4,6-Tris-(terphenyl)-1,3,5-triazin enthält.

## Claims

1. Cosmetic O/W emulsion comprising
a) alkyl sulphate in a concentration of from 0.05 to 1.5% by weight,
b) inorganic UV light protection filter pigments in an amount of at least 10% by weight,
in each case based on the total weight of the preparation,
**characterized in that** the inorganic UV light protection filter pigments used are mixtures of titanium dioxide and zinc oxide pigments,
that the preparation has a light protection factor of at least 15 and that the comprises one or more W/O emulsifiers which are selected from the group of the compounds polysiloxane polyether-polyalkane copolymers, polyglycerol poly-12-hydroxystearate or PEG-30 dipolyhydroxystearate.

2. Cosmetic preparation according to Claim 1, **characterized in that** the emulsion comprises fatty alcohol and/or glycerol monostearate and/or sorbitan stearate in a total concentration of from 0.5 to 8.0% by weight, based on the total weight of the preparation.

3. Cosmetic preparation according to one of the preceding claims, **characterized in that** the ratio of alkyl sulphate to the total amount of inorganic UV light protection filter pigments is from 1:7 to 0.1:60.

4. Cosmetic preparation according to one of the preceding claims, **characterized in that** the alkyl sulphate used is cetostearyl sulphate.

5. Cosmetic preparation according to one of the preceding claims, **characterized in that** the inorganic UV light protection filter pigments are selected from the group of titanium dioxides with a primary particle size between 10 nm to 200 nm and/or of zinc oxides with a primary particle size of < 300 nm.

6. Cosmetic preparation according to one of the preceding claims, **characterized in that** the preparation comprises one or more W/O emulsifiers in a total concentration of from 0.2 to 1.5% by weight, based on the total weight of the preparation.

7. Cosmetic preparation according to one of the preceding claims, **characterized in that** the preparation comprises the particulate organic light
protection filter 2,2'-methylenebis(6-(2H-benzotriazol-2-yl)-4-(1,1,3,3-tetramethylbutyl)phenol).

8. Cosmetic preparation according to one of the preceding claims, **characterized in that** the preparation comprises one or more particulate organic light protection filters is selected from the group 2,4,6-tris(biphenyl)-1,3,5-triazine; 2,4,6-tris(terphenyl)-1,3,5-triazine.

## Revendications

1. Émulsion H/E cosmétique contenant :
a) un sulfate d'alkyle en une concentration de 0,05 à 1,5 % en poids,
b) des pigments inorganiques filtrants protégeant contre la lumière UV en une quantité d'au moins 10 % en poids,
à chaque fois par rapport au poids total de la préparation,
**caractérisée en ce que** des mélanges de pigments de dioxyde de titane et d'oxyde de zinc sont utilisés en tant que pigments inorganiques filtrants protégeant contre la lumière UV,
**en ce que** la préparation présente un indice de protection solaire d'au moins 15, et **en ce que** le contient un ou plusieurs émulsifiants E/H qui sont choisis dans le groupe constitué par les composés copolymères de polysiloxane-polyéther-polyalcane, le poly-12-hydroxystéarate de polyglycérine ou le dipolyhydroxystéarate de PEG-30.

2. Préparation cosmétique selon la revendication 1, caractérisée n ce que l'émulsion contient un alcool gras et/ou du monostéarate de glycérine et/ou du stéarate de sorbitane en une concentration totale de 0,5 à 8,0 % en poids, par rapport au poids total de la préparation.

3. Préparation cosmétique selon l'une quelconque des revendications précédentes, **caractérisée en ce que** le rapport entre le sulfate d'alkyle et la quantité totale de pigments inorganiques filtrants protégeant contre la lumière UV est de 1:7 à 0,1:60.

4. Préparation cosmétique selon l'une quelconque des revendications précédentes, **caractérisée en ce que** du sulfate de cétostéaryle est utilisé en tant que sulfate d'alkyle.

5. Préparation cosmétique selon l'une quelconque des revendications précédentes, **caractérisée en ce que** les pigments inorganiques filtrants protégeant contre la lumière UV sont choisis dans le groupe constitué par les dioxyde de titane d'une taille de particule primaire comprise entre 10 nm et 200 nm et/ou les oxydes de zinc d'une taille de particule primaire < 300 nm.

6. Préparation cosmétique selon l'une quelconque des revendications précédentes, **caractérisée en ce que** la préparation contient un ou plusieurs émulsifiant E/H en une concentration totale de 0,2 à 15 % en poids, par rapport au poids total de la préparation.

7. Préparation cosmétique selon l'une quelconque des revendications précédentes, **caractérisée en ce que** la préparation contient le filtre photoprotecteur organique particulaire 2,2'-méthylène-bis-(6-(2H-benzotriazal-2-yl)-4-(1,1,3,3-tétraméthylbutyl)-phénol.

8. Préparation cosmétique selon l'une quelconque des revendications précédentes, **caractérisée en ce que** la préparation contient un ou plusieurs filtres photoprotecteurs organiques particulaires est choisi dans le groupe constitué par la 2,4,6-tris-(biphényl)-1,3,5-triazine ; la 2,4,6-tris-(terphényl)-1,3,5-triazine.
